**Europäisches Patentamt**

**European Patent Office**

**Office européen des brevets**

(19)

(11) Veröffentlichungsnummer: **0 385 930**
**A1**

(12) **EUROPÄISCHE PATENTANMELDUNG**

(21) Anmeldenummer: **90810095.1**

(22) Anmeldetag: **12.02.90**

(51) Int. Cl.5: **A61F  2/36**

Die Bezeichnung der Erfindung wurde geändert (Richtlinien für die Prüfung im EPA, A-III, 7.3).

(30) Priorität: **02.03.89 CH 780/89**

(43) Veröffentlichungstag der Anmeldung:
**05.09.90 Patentblatt  90/36**

(84) Benannte Vertragsstaaten:
**AT DE FR GB IT**

(71) Anmelder: **GEBRÜDER SULZER AKTIENGESELLSCHAFT**
**Zürcherstrasse 9**

**CH-8401 Winterthur(CH)**

Anmelder: **Protek AG**
**Stadtbachstrasse 64**
**CH-3001 Bern(CH)**

(72) Erfinder: **Spotorno, Lorenzo, Prof. Dr.-med.**
**Ospedale Riunitit**
**I-17024 Finale Ligure(IT)**
Erfinder: **Frey, Otto, Dr.**
**Wallrütistrasse 56**
**CH-8400 Winterthur(CH)**

(54) **Schaft für eine Femurkopfprothese.**

(57) Der Schaft (1) hat im lateralen proximalen Bereich einen nach anterior und posterior von zwei flügelartigen Lappen (14) begrenzten Hohlraum (15). In diesem ist ein Aufspreizkeil (18) vorgesehen, der in Richtung der Schaftachse (6) in den Hohlraum (15) hineingezogen wird; das "Einziehen" des Keiles (18) bewirkt ein Zuganker (3), der dabei seinerseits den durch Schlitze (2) unterteilten Mantel des distalen Kreiszylinders aufspreizt.

Durch das Aufspreizen der Lappen (14) entsteht im proximalen Bereich ein grossflächiger inniger Kontakt zwischen Schaft (1) und Knochen, wodurch zum einen die Bildung von stabilem Knochengewebe und zum anderen eine "weite" Verteilung der Belastungen bei ihrer Weiterleitung in den Knochen entsteht.

Fig.1

EP 0 385 930 A1

## Gerader Schaft für eine Femurkopfprothese

Die Erfindung betrifft einen geraden Schaft für eine Femurkopfprothese, der in seinem distalen Bereich als hohler Kreiszylinder ausgebildet ist, wobei der distale Kreiszylinder mit Längsschlitzen versehen ist und in einen proximalen Teil übergeht, der sich über die Schafthöhe von distal nach proximal allseitig stetig erweitert.

Eine Prothese der vorstehend genannten Art ist bereits vorgeschlagen worden (CH-Patentanmeldung 000 070/89-1). Aufgrund der neuerer Untersuchungen besteht die Tendenz, bei einer zementfreien Verankerung einer Femurkopf-Prothese die Ueberleitung der Belastungen von der Prothese auf den Knochen möglichst grossflächig im proximalen Bereich nahe der Resektionsebene vorzunehmen. Ein besonderes Gewicht wird dabei auf die Zusatzforderung gelegt, dass die als Fremdkörper im Knochen wirkende Prothese in ihrer Elastizität möglichst weitgehend an den Knochen angenähert wird.

Aufgabe der Erfindung ist es, einen Schaft für eine Femurkopf-Prothese zu schaffen, der die vorstehenden Forderungen erfüllt. Gelöst wird diese Aufgabe dadurch, dass der sich im Querschnitt von lateral nach medial verjüngende, proximale Teil lateral einen sich nach distal keilförmig verengenden Hohlraum aufweist, der nach anterior und posterior durch flügelartige Lappen begrenzt ist, und in den ein Aufspreizkeil eingesetzt ist, der seinerseits durch einen Zuganker im distalen Hohlzylinder des Schaftes in den Hohlraum hineingezogen ist.

Die aufspreizbaren Lappen oder Flügel werden bei der Implantation der Prothese durch den Keil, der mit Hilfe des Zugankers in den lateralen Hohlraum hineingezogen wird, so stark gegen das Knochengewebe im Innern des Operationshohlraumes gepresst, dass eine für die Bildung von knöchernem Gewebe erforderliche innige "Verbindung" zwischen Implantat und Knochen grossflächig entsteht. Ueber diese Kontaktfläche werden dabei nach dem Einwachsen von Knochengewebe vor allem Torsionskräfte und ein Teil der Biegebelastungen des Knochens infolge von Momenten weitergegeben. Die relativ dünnen flügelartigen Lappen erhöhen dabei die Elastizität des proximalen Schaftbereichs. Diese Elastizität lässt sich zusätzlich weiter vergrössern, wenn der Ansatz der flügelartigen Lappen an den Schaftkörper teilweise geschlitzt ist, wobei der oder die Schlitze entlang dem nach medial gelegenen Boden des Hohlraumes verlaufen.

Gleichzeitig spreizt der Zuganker, der mit Vorteil konisch ausgebildet ist, das distale Ende des Schaftes so weit auf, dass auch in diesem Bereich Knochengewebe die Verbindung zwischen Schaft und Femur bildet. Im distalen Bereich wird dann ein weiterer Teil der Biegebelastungen, die darüberhinaus noch als Druckbelastungen über die mediale Prothesenschmalseite auf den Calcar-Bogens des Femurs wirken, auf den Knochen übertragen.

Das Anziehen des Aufspreizkeiles - der mit Vorteil aus Kunststoff besteht und dessen offene laterale Begrenzung mit einem Metallgitter, beispielsweise einem Drahtnetz belegt sein kann - lässt sich für den Operateur erleichtern, wenn der Aufspreizkeil im Hohlraum in Richtung der Längsachse des Schaftes geführt ist und/oder wenn der Zuganker mit mindestens einer in den Schlitzen des distalen Hohlzylinders gleitenden Führungsnase versehen ist.

Schliesslich ist es vorteilhaft, wenn für die Aufnahme und Uebertragung der im wesentlichen parallel zur Längsachse des Knochens verlaufenden Druckbelastungen sich der proximale Rand des Hohlraumes in einem den Prothesenhals umgebenden ebenen Kragen fortsetzt, dessen dem Knochen zugewandte Auflageebene mit der Prothesenschaftachse einen Winkel grösser oder gleich $45°$ bis kleiner oder gleich $90°$, vorzugsweise einen Winkel von $75°$, einschliesst.

Im folgenden wir die Erfindung anhand eines Ausführungsbeispiels im Zusammenhang mit der Zeichnung näher erläutert.

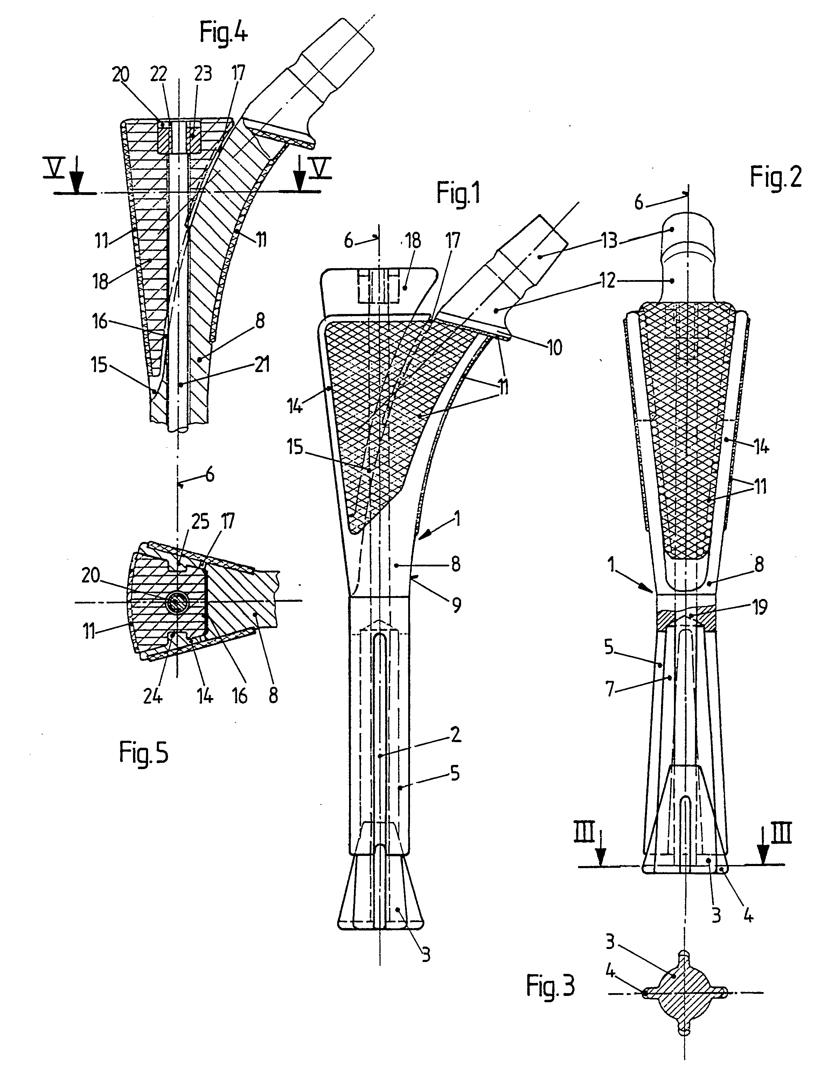

Fig. 1 zeigt schematisch ein Ausführungsbeispiel des neuen Schaftes in einer Ansicht von anterior oder posterior her gesehen, bei dem der Aufspreizkeil und der Zuganker noch nicht "gespannt" sind;

Fig. 2 gibt eine im distalen Bereich als achsparalleler Längsschnitt dargestellte Ansicht von lateral her gesehen wieder, bei der der Aufspreizkeil und Zuganker im "montierten Zustand" dargestellt sind;

Fig. 3 ist ein Schnitt III-III von Fig. 1;

Fig. 4 stellt einen Längsschnitt durch den proximalen Teil des Schaftes dar, während

Fig. 5 der Schnitt V-V von Fig. 4 ist.

Der aus Metall, vorzugsweise aus Titan oder einer Titanlegierung gefertigte Schaft 1 ist in seinem distalen Bereich als hohler Kreiszylinder ausgebildet, in dessen mit der Schaftachse 6 konzentrischen Hohlraum 7 vom distalen Ende her ein konischer Zuganker 3 einsetzbar ist. Durch Schlitze 2, in die Führungsnasen 4 des Zugankers 3 eingreifen, ist der Mantel des Zylinders in aufspreizbare Lappen 5 unterteilt.

An den distalen Kreiszylinder schliesst sich nach proximal ein sich stetig erweiternder proximaler Teil 8 an, dessen mediale Schmalseite 9 in einem Bogen zu einem Kragen 10 führt, der sich bei der implantierten Prothese auf dem Rand des Resektionsschnittes abstützt und vor allem für die Weiterleitung der Druckkräfte von der Prothese auf den Knochen dient. Die Abstützfläche des Kragens 10, die für das Einwachsen von stabilem Knochengewebe mit einer Struktur 11, beispielsweise einem ein oder mehrlagigen Drahtnetz belegt ist, bilden daher mit der Schaftachse 6 einen von distal nach proximal spitzen Winkel zwischen 45° und 90°; vorzugsweise beträgt dieser Winkel beispielsweise 75°.

Auf seiner proximalen Seite trägt der Kragen 10 einen Prothesenhals 12, der in einem Zapfen 13 für die Befestigung eines nicht gezeigten Gelenkkopfes mit Hilfe einer selbsthemmenden Steckverbindung endet.

Der proximale Teil 8 besteht unterhalb des Prothesenhalses 12 und des Kragens 10 aus einem massiven tragfähigen Kern, der nach lateral in zwei flügelartige Lappen 14 übergeht, die nach anterior und nach posterior einen sich nach distal keilförmig verjüngenden Hohlraum 15 begrenzen. Die Lappen 14 sind - wie die Abstützfläche des Kragens 10 und der Bogen der medialen Schmalseite - mit einem, das Einwachsen von Knochengewebe fördernden Drahtnetz 11 belegt.

Entlang dem Bogen 16 des Hohlraumes 15 verlaufen zwischen dem Kern des proximalen Teils 8 und den Flügeln oder Lappen 14 Schlitze 17, durch die die elastische Aufspreizbarkeit der Lappen 14 erhöht wird. Durch mehr oder weniger tiefe Schlitze 17 ist es möglich, den Lappen bei unterschiedlichen Schäften verschiedene Elastizitäten zu geben.

Im Hohlraum 15 ist ein aus Kunststoff gefertigter und lateral wiederum mit einem Drahtnetz 11 belegter Aufspreizkeil 18 gelagert, mit dessen Hilfe die Lappen 14 aufgeweitet werden, so dass zwischen ihrer Auflage 11 und dem sie umgebenden, nicht gezeigten Knochengewebe derart innige Kontakte bestehen, dass stabiles Knochengewebe und nicht nur "weiches" Bindegewebe in die äussere Oberflächenstruktur 11 einwächst. Für das Aufspreizen der Lappen 14 wird der Keil 18 dabei vom Zuganker 3 in den Hohlraum 15 hineingezogen. Durch den proximalen Teil 8 und den Keil 18 verläuft daher vom distalen Hohlraum 7 aus eine mit der Schaftachse 6 koaxiale Bohrung 19, die in einer Ausnehmung des Keils 18 endet. In dieser Bohrung 19 verläuft der Zugstab 21 des Zugankers 3. Der Stab 21 trägt am Ende in der Ausnehmung des Keiles 18 ein Gewinde 22, auf das eine Mutter 23 aufgeschraubt ist, in der Schlitze 20 für ein Einschraubwerkzeug vorgesehen sind.

Für eine geführte Bewegung des Keiles 18 im Hohlraum 15 beim Anziehen der Mutter 23 sind in den Flanken des Keiles 18 Nuten 24 vorgesehen, in die zur Schaftachse 6 parallel Führungsleisten 25 an den Innenseiten der Lappen 14 eingreifen.

Mit dem Anziehen des Zugankers 3 ergeben sich für die Weiterleitung der Belastungen von der Prothese auf den Knochen grossflächige Kontaktbereiche zwischen Knochen und Prothese im lateralen proximalen Bereich, wobei die flügelartigen Lappen in ihrer Elastizität weitgehend an die Knochenelastizität angenähert sind. Das gleichzeitige Aufspreizen des geschlitzten distalen Zylinders bewirkt vor allem, dass die nach lateral wirkenden Druckkräfte infolge der Biegemomente ebenfalls relativ grossflächig in den Knochen weitergeleitet werden.

## Ansprüche

1. Gerader Schaft für eine Femurkopfprothese, der in seinem distalen Bereich als hohler Kreiszylinder ausgebildet ist, wobei der distale Kreiszylinder mit Längsschlitzen versehen ist und in einen proximalen Teil übergeht, der sich über die Schafthöhe von distal nach proximal allseitig stetig erweitert, dadurch gekennzeichnet, dass der sich im Querschnitt von lateral nach medial verjüngende, proximale Teil (8) lateral einen sich nach distal keilförmig verengenden Hohlraum (15) aufweist, der nach anterior und posterior durch flügelartige Lappen (14) begrenzt ist, und in den ein Aufspreizkeil (18) eingesetzt ist, der seinerseits durch einen Zuganker (3) im distalen Hohlzylinder (7) des Schaftes (1) in den Hohlraum (15) hineingezogen ist.

2. Schaft nach Anspruch 1 dadurch gekennzeichnet, dass der Aufspreizkeil (18) im Hohlraum (15) in Richtung der Längsachse (6) des Schaftes (1) geführt ist.

3. Schaft nach Anspruch 1 oder 2, dadurch gekennzeichnet, dass der Ansatz der flügelartigen Lappen (14) an den Schaftkörper (8) teilweise geschlitzt ist, wobei der oder die Schlitze (17) entlang dem nach medial gelegenen Boden (16) des Hohlraumes (15) verlaufen.

4. Schaft nach einem der Ansprüche 1 bis 3, dadurch gekennzeichnet, dass sich der proximale Rand des Hohlraumes (15) in einem den Prothesenhals (12) umgebenden, ebenen Kragen (10) fortsetzt, dessen dem Knochen zugewandte Abstützebene mit der Längsachse (6) des Schaftes (1) einen Winkel grösser/gleich 45° bis kleiner/gleich 90° einschliesst.

5. Schaft nach einem der Ansprüche 1 bis 4 dadurch gekennzeichnet, dass der Zuganker (3) mit mindestens einer in den Schlitzen (2) des distalen Hohlzylinders (7) gleitenden Führungsnase (4) versehen ist.

6. Schaft nach einem der Ansprüche 1 bis 5, dadurch gekennzeichnet, dass der Aufspreizkeil (18) aus Kunststoff besteht, und dass seine offene, laterale Begrenzung mit einem Metallgitter (11), beispielsweise einem Drahtnetz, belegt ist.

7. Schaft nach Anspruch 4, dadurch gekennzeichnet, dass der Winkel zwischen der Abstützfläche des Kragens (10) und der Längsachse (6) 75° beträgt.

Fig.4

Fig.1

Fig.2

Fig.5

Fig.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 520 511 (P. GIANEZIO & G. NUNZIO) <br> * Spalte 1, Zeile 66 - Spalte 2, Zeile 19; Figuren 1,2 * <br> --- | 1,4,7 | A 61 F 2/36 |
| A | US-A-4 681 590 (J. TANSEY) <br> * Spalte 3, Zeile 16 - Spalte 4, Zeile 5; Figuren 1-5 * <br> --- | 1,2,4 | |
| A | WO-A-8 801 492 (OFFICE MEDICO CHIRURGICAL INTERNATIONAL) <br> * Seite 2, Zeile 30 - Seite 3, Zeile 6; Seite 3, Zeilen 18-35; Figuren 1,2,4 * <br> --- | 1,2 | |
| A | DE-B-2 247 560 (A. FISCHER) <br> * Ansprüche 1,2,9; Spalte 6, Zeilen 43-49; Figur 1 * <br> --- | 1,5 | |
| A | EP-A-0 234 358 (GEBRÜDER SULZER) <br> * Zusammenfassung; Figur 1 * <br> ----- | 6 | |
| | | | **RECHERCHIERTE SACHGEBIETE (Int. Cl.5)** <br><br> A 61 F |

Der vorliegende Recherchenbericht wurde für alle Patentansprüche erstellt

| Recherchenort | Abschlußdatum der Recherche | Prüfer |
|---|---|---|
| DEN HAAG | 18-05-1990 | NICE P.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument